# DEMANDE DE BREVET EUROPEEN

(11) **EP 0 862 922 A1**
(43) Date de publication de la demande: **09.09.1998**
(21) Numéro de dépôt: 98400413.5
(22) Date de dépôt: 20.02.1998
(51) Int. Cl.: A61M 16/00

(54) **Appareil d'assistance respiratoire muni d'un dispositif pour créer une dépression dans le circuit expiratoire**

(30) Priorité: 03.03.1997 FR 9702502
(71) Demandeur: Société d'Applications Industrielles Medicales et Electroniques ( SAIME), 77176 Savigny Le Temple (FR)
(72) Inventeur: Chalvignac, Philippe, 77123 Noisy sur Ecole (FR); Dehour, Patrick, 77000 Melun (FR)
(74) Mandataire: Kohn, Philippe

(57) **Abrégé**

L'invention propose un appareil d'assistance respiratoire, du type comportant une source d'air sous pression (16) qui fournit de l'air sous pression destiné à être acheminé, lors d'une phase inspiratoire, vers un patient au travers d'un circuit inspiratoire (12), et du type dans lequel de l'air expiré par le patient au cours d'une phase expiratoire est recueilli par un circuit expiratoire (14),

caractérisé en ce que l'appareil (10) comporte des moyens (58) pour relier le circuit expiratoire (14) à une source de dépression (56) pendant au moins une partie de la phase expiratoire.

## Description

L'invention concerne un appareil d'assistance respiratoire muni d'un dispositif pour créer une dépression dans le circuit expiratoire.

L'invention concerne plus particulièrement un appareil d'assistance respiratoire, du type comportant une source d'air sous pression qui fournit de l'air sous pression destiné à être acheminé, lors d'une phase inspiratoire, vers un patient au travers d'un circuit inspiratoire, et du type dans lequel de l'air expiré par le patient au cours d'une phase expiratoire est recueilli par un circuit expiratoire.

Il existe de nombreux types d'appareils d'assistance respiratoire, aussi appelés respirateurs, qui permettent de pallier aux troubles de la respiration en procurant notamment au patient une aide insufflatoire lors des phases inspiratoires.

Le patient porte généralement sur le visage un masque qui recouvre le nez et la bouche et auquel le circuit inspiratoire est relié de manière à forcer l'inspiration du patient.

Toutefois, dans un tel type d'appareil, l'expiration du patient doit se faire obligatoirement au travers du masque et il en résulte dans certains cas une gêne pour le patient, notamment en début de phase expiratoire lorsque le débit expiratoire est maximal.

Cette gêne est plus particulièrement sensible lorsque la vanne expiratoire, au travers de laquelle le patient expire vers l'atmosphère, est agencée non pas directement au niveau du masque mais à une certaine distance de celui-ci.

En effet, la vanne expiratoire est parfois éloignée du visage du patient pour que l'expiration ne se fasse pas directement à côté de son visage. Dans certains appareils, il est même prévu que la vanne expiratoire soit agencée au niveau de l'appareil d'assistance respiratoire.

En effet, les gaz expiratoires sont alors ramenés vers l'appareil par une conduite expiratoire, séparée de la conduite inspiratoire, de sorte qu'il est possible de procéder à des analyses de débit et de pression du flux expiratoire, voire même de procéder à une analyse de la composition des gaz expirés par des capteurs qui sont alors implantés directement dans l'appareil et non plus à distance, ce qui simplifie considérablement l'intégration de ces capteurs dans un circuit de contrôle du respirateur.

L'inconvénient de ce type de réalisation, dite à circuit double, est que les pertes de charge dans le circuit expiratoire sont relativement élevées et augmentent encore l'effort que le patient doit fournir pour expirer, notamment en début d'expiration lorsque le débit expiratoire est maximum.

L'invention a donc pour objet de proposer un appareil d'assistance respiratoire qui comporte des moyens permettant de réduire l'effort que doit fournir le patient lors de la phase expiratoire pour expulser les gaz expirés, notamment lors de l'utilisation d'un circuit double.

A cet effet, l'invention propose un appareil d'assistance respiratoire du type décrit précédemment, caractérisé en ce que l'appareil comporte des moyens pour relier le circuit expiratoire à une source de dépression pendant au moins une partie de la phase expiratoire.

Selon d'autres caractéristiques de l'invention :
- le circuit expiratoire est relié à la source de dépression en début de phase expiratoire ;
- le circuit expiratoire comporte une chambre expiratoire qui est agencée dans l'appareil, et une conduite expiratoire qui relie la chambre expiratoire au patient, et la chambre expiratoire est reliée d'une part à une sortie des gaz expirés par une vanne expiratoire, et d'autre part à la source de dépression par une vanne de dépression ;
- un capteur de débit est interposé dans le circuit expiratoire entre la conduite expiratoire et la chambre expiratoire ;
- le circuit inspiratoire comporte une chambre inspiratoire qui est agencée dans l'appareil et dans laquelle débouche la source d'air sous pression, et une conduite inspiratoire qui relie la chambre inspiratoire au patient, et la chambre inspiratoire est reliée d'une part à la conduite inspiratoire au travers d'une vanne inspiratoire, et d'autre part à une sortie d'échappement par une vanne d'échappement ;
- les vannes inspiratoire, expiratoire, d'échappement, et de dépression sont réalisées sous la forme de vannes à membranes à commande pneumatique, et elles sont agencées dans un bloc de distribution dans lequel sont formées les chambres inspiratoire et expiratoires ;
- l'une au moins des vannes à commande pneumatique est aménagée sous la forme d'une empreinte creusée dans le bloc de distribution, l'empreinte débouche vers l'extérieur dans une face latérale du bloc et comporte, dans le fond, une enceinte interne et une enceinte périphérique aménagée autour de l'enceinte interne, les deux enceintes sont délimitées l'une de l'autre par une paroi dont une extrémité s'étend vers l'extérieur sous la forme d'un tronçon tubulaire qui se termine par une face annulaire transversale tournée vers l'extérieur et formant siège d'étanchéité, en ce qu'une membrane transversale souple est liée de manière étanche par son bord périphérique à une paroi de l'empreinte du bloc, vers l'extérieur par rapport au siège d'étanchéité, pour séparer les deux enceintes d'un logement de commande, et une surpression dans le logement de commande est susceptible de plaquer la membrane en appui contre le siège d'étanchéité pour isoler de manière étanche l'enceinte interne de l'enceinte périphérique et ainsi fermer la vanne en interrompant la communication entre les deux enceintes ;
- le logement de commande est délimité vers l'extérieur par une face interne d'un capot distributeur, une conduite d'alimentation du logement de commande est aménagée dans le bloc de distribution et débouche dans une surface périphérique de l'empreinte, en regard d'une gorge qui est aménagée dans une surface périphérique du capot distributeur, et le capot comporte un canal qui débouche d'une part dans la gorge et d'autre part dans la face avant pour relier la conduite d'alimentation au logement de commande ;
- le bloc de distribution est monté de manière amovible dans l'appareil ;
- la source de pression est un moto-ventilateur, et la source de dépression est constituée par une entrée d'aspiration du moto-ventilateur ;
- la source de pression est un moto-ventilateur de type centrifuge ;
- le capteur de débit comporte un corps externe dans lequel est engagée une douille tubulaire qui délimite un canal d'écoulement de l'air, la douille comporte deux séries de perçages radiaux qui débouchent d'une part vers l'intérieur, respectivement de part et d'autre axialement d'un corps poreux interposé dans le canal d'écoulement, et d'autre part vers l'extérieur respectivement dans deux gorges annulaires aménagées dans une surface cylindrique externe de la douille, et le corps externe comporte deux orifices qui débouchent chacun en regard d'une des gorges et qui sont reliés à des capteurs de pression ;
- les orifices de prise de pression du corps externe ne débouchent pas en regard d'un perçage radial de la douille.

D'autres caractéristiques et avantages de l'invention apparaîtront à la lecture de la description détaillée qui suit pour la compréhension de laquelle on se reportera aux dessins annexés dans lesquels :
- les figures 1 à 3 sont des schémas illustrant le principe de fonctionnement d'un mode de réalisation de l'invention, respectivement en phase inspiratoire, en phase de dépression dans le circuit expiratoire, et en phase expiratoire ;
- la figure 4 est une vue en perspective éclatée illustrant un mode de réalisation du bloc de distribution d'un appareil d'assistance respiratoire selon l'invention ;
- la figure 5 est une vue agrandie d'un détail de la figure 1 illustrant plus particulièrement des vannes à commande pneumatique du bloc de distribution ;
- la figure 6 est une vue schématique en perspective avec arrachement illustrant la géométrie interne d'une vanne à commande pneumatique ;
- la figure 7 est une vue de côté illustrant l'aménagement de la vanne pneumatique dans le bloc de distribution ;
- la figure 8 est une vue en coupe illustrant la vanne à commande pneumatique ;
- les figures 9, 10 et 11 sont des vues en coupe selon la ligne 9-9 de la figure 7 du bloc de distribution de l'appareil selon l'invention, représenté respectivement en phase inspiratoire, en phase de dépression et en phase expiratoire ;
- les figures 9A, 10A, 11A sont des vues en coupe selon la ligne A-A respectivement des figures 9, 10, 11 ;
- les figures 9B, 10B, 11B sont des vues en coupe selon la ligne B-B respectivement des figures 9, 10, 11 ;
- la figure 12 est une vue en coupe axiale d'un capteur de l'appareil d'assistance respiratoire selon l'invention ;
- la figure 13 est une vue en perspective illustrant la structure générale d'un appareil selon l'invention ; et
- la figure 14 est une vue similaire à celle de la figure 1 illustrant un deuxième mode de réalisation de l'invention.

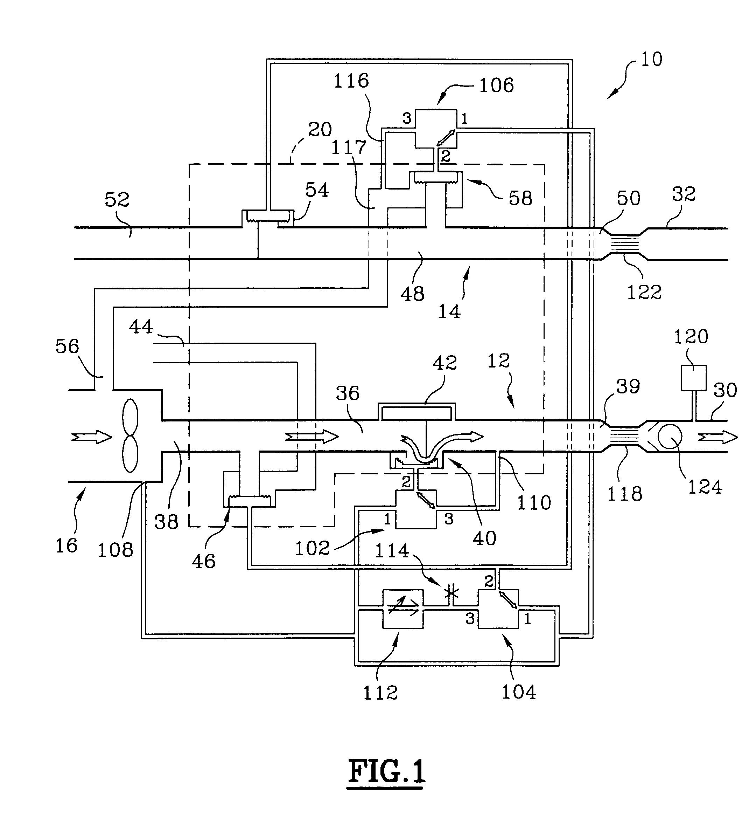

On a représenté sur les figures 1 à 3 un schéma illustrant de manière simplifiée la structure d'un appareil d'assistance respiratoire 10, ou respirateur, selon l'invention.

L'appareil 10 comporte un circuit inspiratoire 12 qui est destiné à acheminer de l'air sous pression à un patient au cours de phases respiratoires. Lors de phases expiratoires, la fourniture d'air sous pression est arrêtée et les gaz expirés par le patient sont recueillis dans un circuit expiratoire 14 avant d'être rejetés dans l'atmosphère.

Dans l'exemple de réalisation qui est décrit ci-après, l'appareil 10 est un appareil autonome, plus particulièrement destiné à être utilisé au domicile du malade de sorte qu'il comporte sa propre source de production d'air sous pression. Ici, l'appareil 10 comporte ainsi un moto-ventilateur 16 qui est avantageusement réalisé sous la forme d'un compresseur centrifuge et qui peut être alimenté par exemple grâce au courant du secteur ou grâce à des batteries d'accumulateur (non représentées).

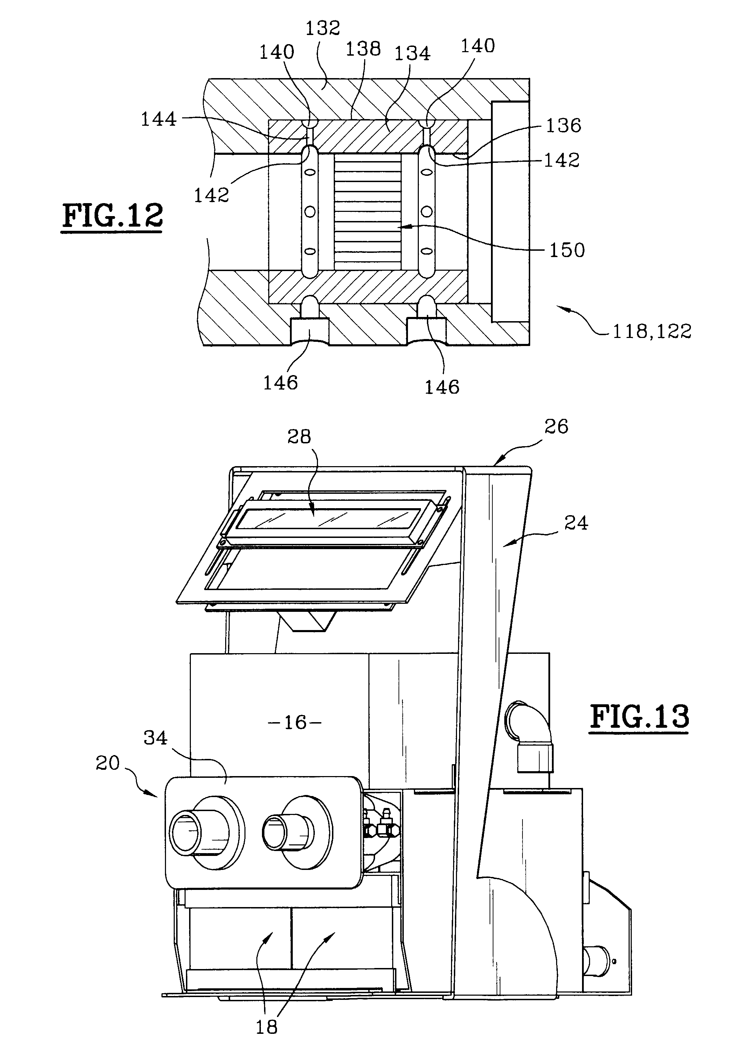

Un tel appareil d'assistance respiratoire 10 doit donc être particulièrement compact. Un exemple de réalisation de l'appareil 10 est représenté de manière schématique à la figure 13 sur laquelle on peut voir que sont empilés verticalement les batteries d'accumulateur 18, un bloc de distribution 20, et un compresseur centrifuge 16 avec ses moyens d'entraînement. L'appareil selon l'invention est d'ailleurs prévu pour pouvoir fonctionner de manière autonome grâce à l'énergie de ses seules batteries 18 pendant une longue période.

L'appareil 10 est ici représenté sans sa carrosserie d'habillage de manière à laisser apparaître une armature verticale 24 qui porte, à son extrémité supérieure 26 formant anse de transport, un afficheur à cristaux liquides 28 destiné à faciliter la programmation du respirateur 10, et plus particulièrement celle d'un circuit de commande (non représenté) qui est porté par une plaque à circuits imprimés susceptible d'être agencée en arrière de l'afficheur 28.

Le circuit de commande est destiné à commander le fonctionnement du moto-ventilateur 16, du bloc de distribution 20 et, de manière générale, l'ensemble du respirateur 10.

Pendant le fonctionnement du respirateur 10, le patient porte généralement sur le visage un masque (non représenté) qui recouvre la bouche et le nez et qui est relié au bloc de distribution 20 par une conduite inspiratoire 30.

Par ailleurs, l'appareil d'assistance respiratoire 10 selon l'invention est plus particulièrement prévu pour être avantageusement utilisé avec un masque comportant une conduite expiratoire 32, indépendante de la conduite inspiratoire 30, pour recueillir et ramener vers l'appareil 10 les gaz expirés par le patient.

Comme on peut le voir sur la figure 13, les conduites inspiratoire 30 et expiratoire 32 sont destinées à être branchées sur une face avant 34 du bloc de distribution 20.

Les conduites inspiratoire 30 et expiratoire 32 sont avantageusement réalisées sous la forme de tubes flexibles et il est possible de les réaliser au moins sur une partie de leur longueur sous la forme de deux tubes coaxiaux dont l'un est entièrement compris à l'intérieur de l'autre.

Comme on peut le voir plus particulièrement sur les figures 1 à 3 et 9 à 11, le circuit inspiratoire 12 comporte une chambre inspiratoire 36 qui est aménagée dans le bloc de distribution 20. Une extrémité longitudinale amont 38 de la chambre inspiratoire 36 est reliée à une sortie d'air du moto-ventilateur 16 tandis qu'une extrémité longitudinale aval 39 débouche dans la conduite inspiratoire 30.

La chambre inspiratoire 36 est munie d'une vanne inspiratoire 40 à commande pneumatique qui est agencée de manière à permettre l'interruption de la circulation d'air dans la chambre inspiratoire 36 entre l'amont 38 et l'aval 39. Toutefois, une dérivation calibrée 42 contourne la vanne inspiratoire 40 de manière à permettre la circulation d'un flux d'air de débit minimal, appelé débit de fuite, dans le circuit inspiratoire 12 même lorsque la vanne inspiratoire 40 est fermée.

En amont de la vanne inspiratoire 40, la chambre inspiratoire 36 est reliée à une sortie d'échappement d'air 44 au travers d'une vanne d'échappement 46, elle aussi à commande pneumatique.

De manière symétrique, le circuit expiratoire 14 comporte une chambre expiratoire 48, qui s'étend longitudinalement parallèlement à la chambre inspiratoire 36 dans le bloc de distribution 20, qui est reliée à l'amont 50 à la conduite expiratoire 32 et qui débouche à l'aval 52 vers une sortie des gaz expirés, au travers d'une vanne expiratoire 54 à commande pneumatique.

Conformément aux enseignements de l'invention, l'appareil d'assistance respiratoire 10 comporte des moyens qui permettent de relier le circuit expiratoire 14 à une source de dépression pendant au moins une partie de la phase expiratoire.

A cet effet, la chambre expiratoire 48 est reliée, entre son extrémité amont 50 et la vanne expiratoire 54, à une entrée 56 d'aspiration d'air du moto-ventilateur 16, au travers d'une vanne de dépression 58 à commande pneumatique.

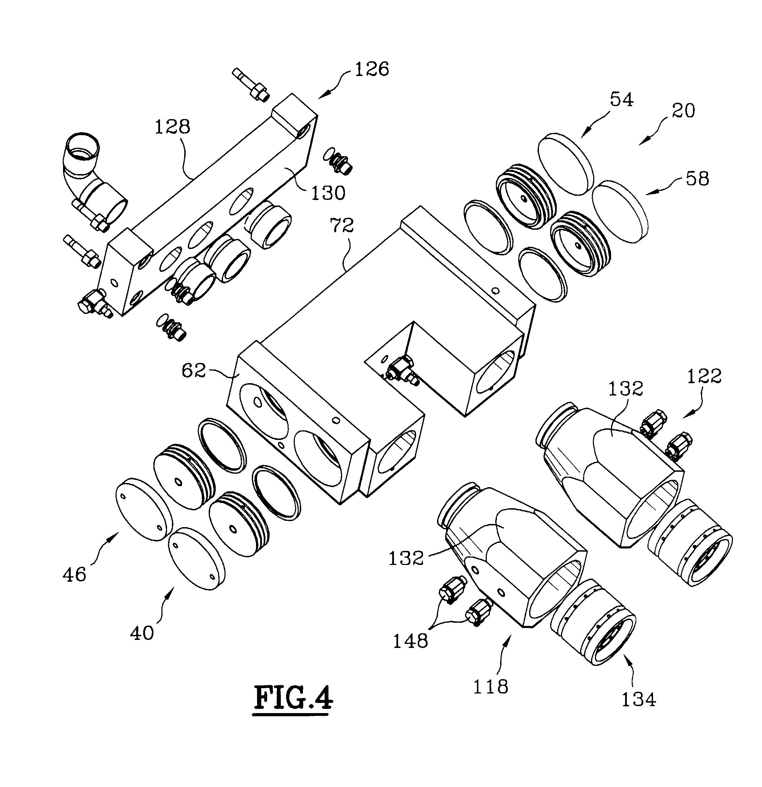

Avantageusement, les quatre vannes d'échappement 46, inspiratoire 40, expiratoire 54 et de dépression 58 sont regroupées dans le bloc de distribution 20 qui, comme on peut le voir sur les figures 4 et 9 à 11, est particulièrement compact et présente l'avantage de pouvoir être facilement démonté de l'appareil en vue de son nettoyage et de sa stérilisation.

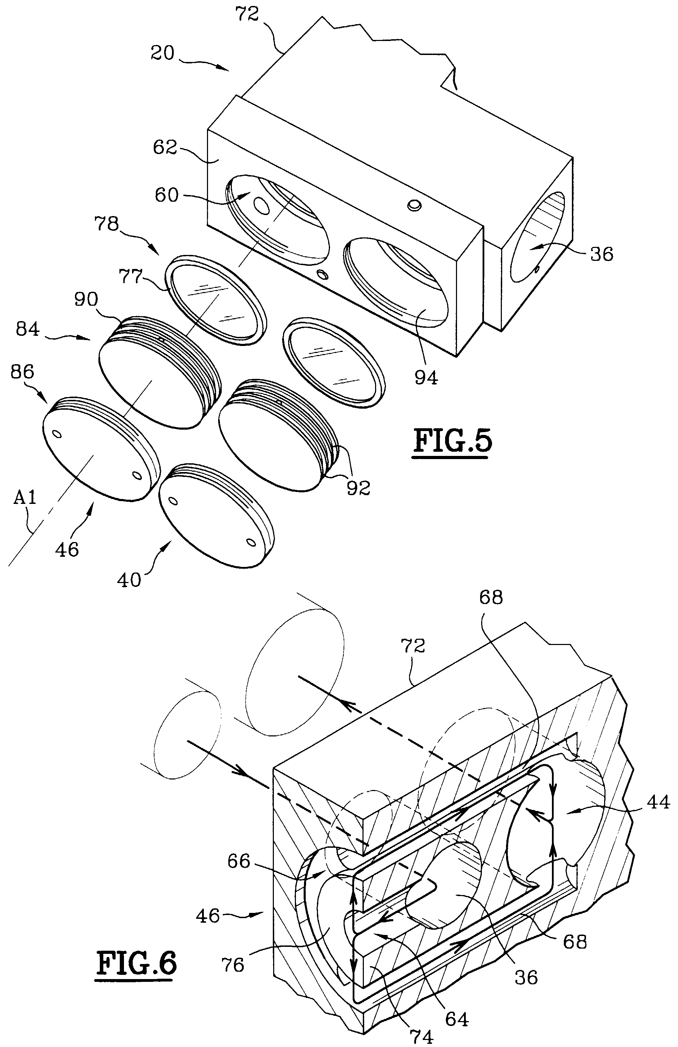

Les quatre vannes commande pneumatique sont toutes réalisées selon le même principe de déformation d'une membrane souple formant élément d'étanchéité. En effet, tout comme les chambres inspiratoire 36 et expiratoire 48, elles sont réalisées venues de matière dans la masse du bloc de distribution 20, et elles sont ainsi similaires à la vanne d'échappement 46 qui sera plus particulièrement décrite en référence aux figures 5 à 8.

La vanne d'échappement 46 est réalisée sous la forme d'une empreinte 60 qui est creusée dans le bloc de distribution 20, selon une direction axiale A1 perpendiculaire à la direction générale longitudinale des chambres inspiratoire 36 et expiratoire 48, et qui débouche vers l'extérieur dans une face latérale longitudinale 62 du bloc 20.

L'empreinte 60 délimite, axialement au fond vers l'intérieur du bloc 20, une enceinte interne cylindrique axiale 64 qui débouche vers l'intérieur dans la chambre inspiratoire 36. Autour de l'enceinte interne 64 est aménagée une chambre périphérique annulaire 66. Des galeries 68 s'étendent axialement vers l'intérieur depuis le fond de l'enceinte annulaire périphérique 66 pour déboucher dans la sortie d'échappement 44 qui est réalisée sous la forme d'un alésage cylindrique qui s'étend parallèlement à la chambre inspiratoire 36, et qui débouche longitudinalement vers l'arrière dans une face arrière 72 du bloc de distribution 20.

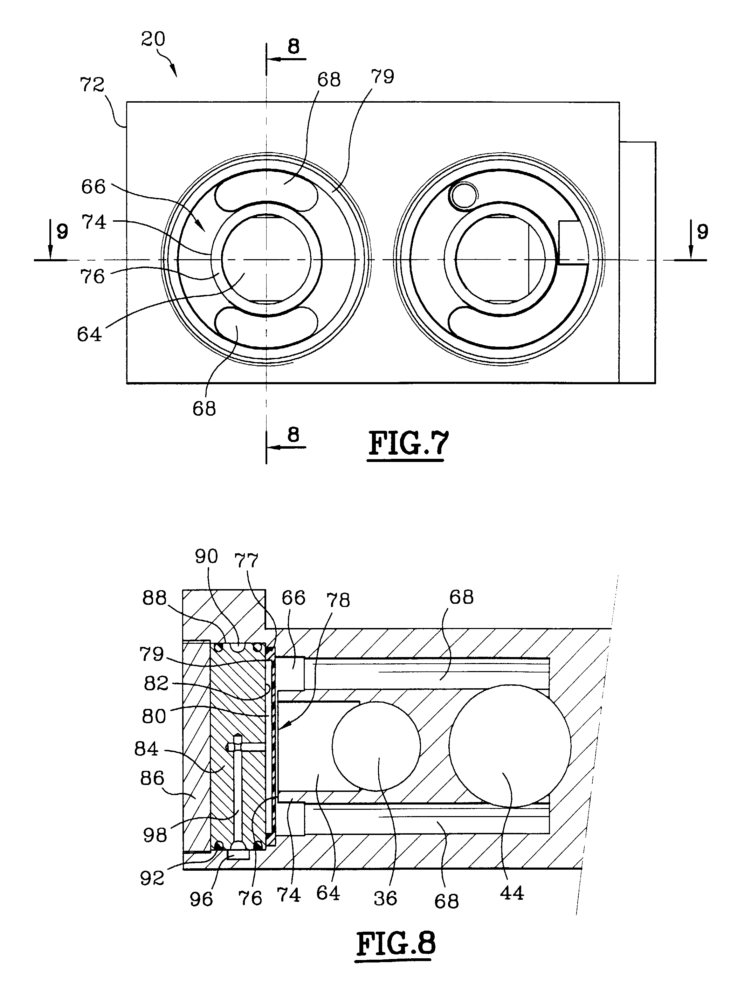

Comme on peut le voir plus particulièrement sur les figures 6 et 7, les galeries 68 présentent, en coupe selon un plan perpendiculaire à la direction axiale A1 de la vanne d'échappement 46, une forme de haricot et elles sont diamétralement opposées de part et d'autre de l'axe A1 de la vanne 46 pour passer respectivement au dessus et au dessous de la chambre inspiratoire 36.

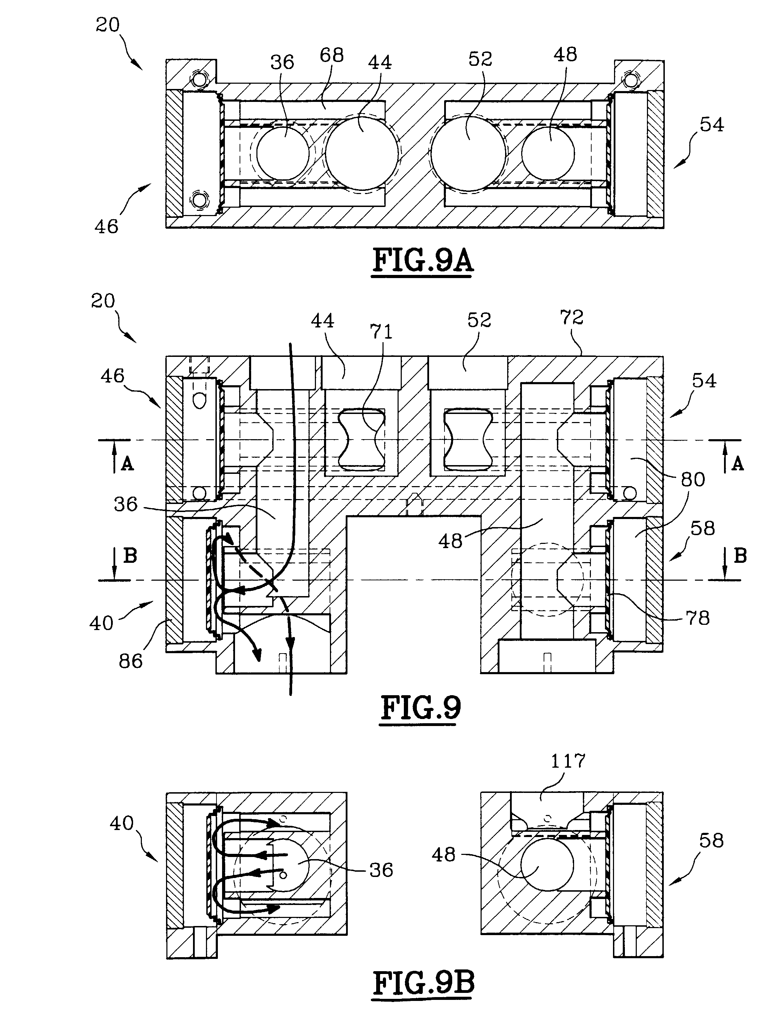

En vue de dessus, l'intersection 71 des galeries 68 avec la sortie d'échappement 44 présente sensiblement la forme d'un diabolo, comme on peut le voir plus particulièrement sur les figures 9 à 11.

Dans l'empreinte 60, les enceintes interne 64 et périphérique 66 sont séparées par une paroi tubulaire axiale 74 dont une face annulaire d'extrémité 76, tournée vers l'extérieur du bloc 20, est destinée à former un siège d'étanchéité de la vanne 46.

Une membrane déformable 78 est agencée en travers de l'empreinte 60, axialement vers l'extérieur de celle-ci par rapport à la paroi tubulaire 74. La membrane 78 comporte en effet un bord périphérique 77 qui est en appui de manière étanche contre un épaulement 79 formé dans une surface cylindrique 94 de l'empreinte 60.

La membrane 78 sépare les deux enceintes 64, 66 par rapport à un logement de commande 80 qui s'étend du côté extérieur de la membrane 78 et qui est délimité axialement vers l'extérieur par la face avant 82 d'un capot distributeur 84 engagé de manière étanche dans une partie extérieure de l'empreinte 60. Comme on peut le voir sur la figure 8, le capot distributeur 84 est maintenu en place à l'intérieur de l'empreinte 60 par un obturateur fileté 86 qui est vissé dans l'extrémité débouchante de l'empreinte 60 de manière à affleurer sensiblement au niveau de la face externe 62 du bloc de distribution 20.

Le logement de commande 80 est destiné à être alimenté en air sous pression de manière à forcer la membrane 78 en appui vers l'intérieur, par une partie centrale, contre la surface annulaire 76 de la paroi tubulaire 74 qui forme siège d'étanchéité. Dans cette position, la membrane 78 interdit toute communication entre les deux enceintes interne 64 et périphérique 66 de sorte qu'il n'y a pas de communication possible entre la chambre inspiratoire 36 et la sortie d'échappement 44.

Au contraire, lorsque l'alimentation en air sous pression du logement de commande 80 est coupée, il existe un jeu suffisant entre la membrane 78 et le siège d'étanchéité 76 pour permettre à un flux d'air de circuler de l'enceinte interne 64 vers l'enceinte périphérique 66, ou inversement, mettant ainsi en communication la chambre inspiratoire 36 et la sortie d'échappement 44.

Le capot distributeur 84 est réalisé sous la forme d'un galet cylindrique d'axe A1 dans lequel on a aménagé, sur une surface cylindrique externe 88, une gorge annulaire collectrice 90 qui est intercalée axialement entre deux gorges 92 destinées à recevoir des joints toriques d'étanchéité susceptibles de coopérer avec la paroi cylindrique interne 94 de l'empreinte 60.

Un réseau de distribution d'air de commande sous pression, qui est aménagé dans le bloc de distribution 20, comporte une conduite 96 qui débouche dans la paroi cylindrique interne 94 de l'empreinte 60 en regard de la gorge collectrice 90 du capot distributeur 84. Le capot distributeur 84 comporte également un canal interne 98 qui comporte un tronçon sensiblement radial et un tronçon sensiblement axial, concourants, et qui débouchent respectivement dans la gorge collectrice 90 et dans la face avant 82 du capot distributeur 84.

Ainsi, de l'air sous pression amené par la conduite 96 est conduit jusqu'au logement de commande 80 pour commander l'ouverture ou la fermeture de la vanne 46.

Comme on peut le voir sur les figures 1 à 3, les vannes à commande pneumatique 40, 46, 54, 58 sont commandées par des électrovannes 102, 104, 106 à trois entrées 1, 2 et 3 (référencées par la suite 102-1, 102-2 106-3) et à deux positions référencées 2-1 et 2-3 en fonction des entrées qui sont mises en communication.

L'électrovanne 102 qui commande la vanne inspiratoire 40 est reliée par une entrée 102-2 au logement de commande de la vanne 40, et par une entrée 102-1 à une prise de pression 108 agencée au niveau de la sortie d'air du moto-ventilateur 16 de telle sorte que lorsque l'électrovanne 102 est en position 2-1, la pression fournie par le moto-ventilateur 16 est fournie au logement de commande 80 de la vanne à commande pneumatique 40 et provoque la fermeture de celle-ci.

L'électrovanne 102 est aussi reliée par son entrée 102-3 à une prise de pression 110 qui débouche dans la chambre inspiratoire 36, en aval de la vanne inspiratoire 40.

Lorsque l'électrovanne 102 est en position 2-3, la vanne inspiratoire 40 est ouverte et il se produit un phénomène d'autorégulation du flux d'air traversant la vanne inspiratoire 40. En effet, une chute de pression en aval de la vanne 40 provoque une diminution de la pression dans le logement de commande 80 de la vanne 40 de sorte que son degré d'ouverture augmente et que le débit d'air au travers de la vanne 40 augmente et tend à provoquer une augmentation de la pression en aval de la vanne 40. Au contraire, si la pression en aval de la vanne inspiratoire 40 augmente, la pression dans le logement de commande 80 augmente aussi, ce qui réduit le débit au travers de la vanne 40 et ce qui tend donc à diminuer la pression en aval de la vanne 40.

Comme on peut le voir sur les figures 1 à 3, la vanne d'échappement 46 et la vanne expiratoire 54 sont commandées par une même électrovanne 104 dont une entrée 104-2 est reliée au logement de commande 80 des deux vannes 46, 54. Cette électrovanne 104 est reliée par son entrée 104-1 à la prise de pression 108 en sortie du moto-ventilateur 16 de telle sorte que lorsque l'électrovanne 104 est en position 2-1, les vannes d'échappement 46 et expiratoire 54 sont fermées.

L'entrée 104-3 de l'électrovanne 104 de commande des vannes d'échappement 46 et expiratoire 54 est reliée à la sortie d'une électrovanne proportionnelle 112 dont l'entrée est reliée à la prise de pression 108 en sortie de moto-ventilateur 16. Une fuite calibrée 114 est agencée entre l'électrovanne proportionnelle 112 et l'entrée 104-3 de l'électrovanne 104. En modulant le degré d'ouverture de la vanne proportionnelle 112, il est ainsi possible de moduler la pression d'air de commande qui est fournie au niveau de l'entrée 104-3, et donc de commander avec précision la pression régnant dans le logement de commande 80 des vannes 46, 54 lorsque l'électrovanne 104 est en position 2-3.

Ce dispositif permet notamment de régler de manière fine le débit susceptible de s'écouler au travers des vannes d'échappement 46 et expiratoire 54. Toutefois, en variante, on peut remplacer l'électrovanne proportionnelle par un compresseur additionnel commandé pour fournir une pression adéquate.

Enfin, les entrées 106-1, 106-2, et 106-3 de l'électrovanne 106 de commande de la vanne de dépression 58 sont reliées, de manière similaire à celles de l'électrovanne 102 de commande de la vanne inspiratoire 40, de telle sorte que lorsque l'électrovanne 106 est en position 1-2, la vanne de dépression 58 est fermée tandis qu'elle est ouverte lorsque l'électrovanne est en position 2-3. Lorsque la vanne de dépression 58 est ouverte, il s'effectue une autorégulation du débit au travers de la vanne de dépression 58 grâce à une dérivation 116 qui relie l'entrée 106-3 de l'électrovanne 106 à l'aval 117 de la vanne de dépression 58.

Le fonctionnement du respirateur 10 selon l'invention sera maintenant plus particulièrement décrit en référence aux figures schématiques 1 à 3 et aux figures 9 à 11, 9A à 11A, et 9B à 11B dans lesquelles le bloc de distribution 20 est représenté de manière plus précise mais dans lesquelles les vannes à commande pneumatique 40, 46, 54, 58 sont représentées de manière schématique pour plus de clarté.

Lorsque le patient est en phase inspiratoire, le respirateur 10, et notamment le bloc de distribution 20, est dans l'état représenté aux figures 1, 9A, et 9B.

Dans cet état, seule la vanne inspiratoire 40 est ouverte tandis que les vannes d'échappement 46, expiratoire 54 et de dépression 58 sont fermées. De la sorte l'air fourni par le moto-ventilateur 16 s'écoule au travers de la chambre inspiratoire 36, au travers de la vanne inspiratoire 40, pour être fourni au patient par l'intermédiaire de la conduite inspiratoire 30.

Un capteur de débit 118, interposé entre la chambre inspiratoire 36 et la conduite inspiratoire 30, et un capteur de pression 120, agencé dans la conduite inspiratoire 30, sont reliés à un circuit de commande du respirateur 20 qui permet notamment de commander la vitesse de rotation du moto-ventilateur 16 afin de délivrer au patient un volume d'air connu sous une pression connue.

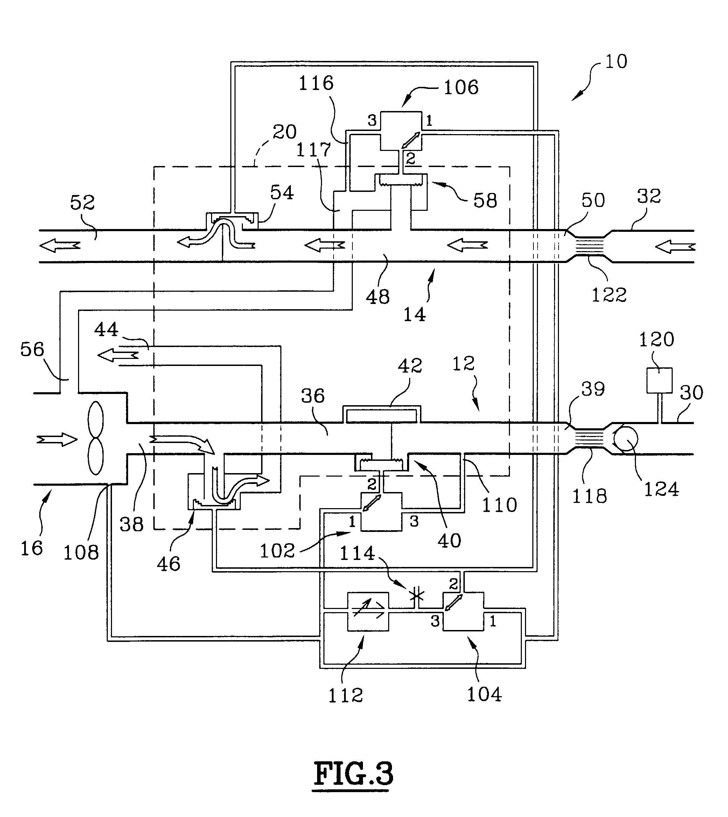

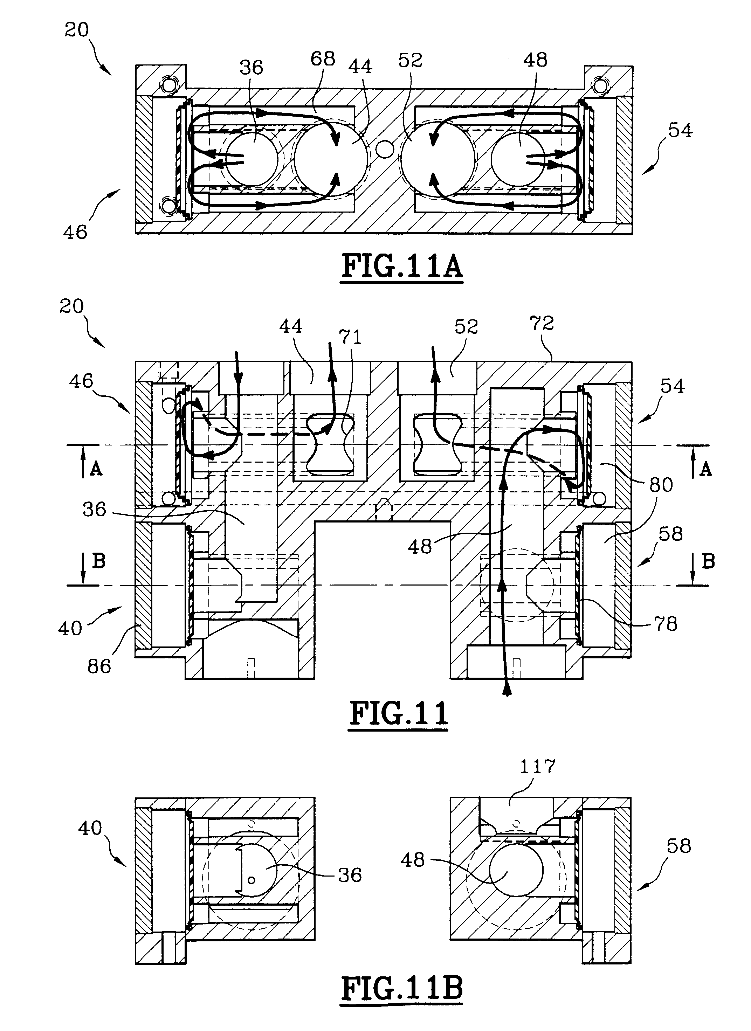

Comme on peut le voir sur les figures 3, 11, 11A et 11B, lorsque le patient est en phase expiratoire, les vannes inspiratoire 40 et de dépression 58 sont fermées tandis que les vannes d'échappement 46 et expiratoires 54 sont ouvertes.

Ainsi, l'air sous pression fourni par le moto-ventilateur 16 est directement évacué au travers de la vanne d'échappement 46 en direction de la sortie d'échappement 44.

Le circuit inspiratoire 12 est obturé par la vanne inspiratoire 40. Un clapet anti-retour 124, agencé dans le circuit inspiratoire 12 entre le débitmètre 118 et le capteur de pression 120, empêche les gaz expirés de retourner vers la chambre inspiratoire 36. On peut toutefois choisir de ne pas installer de clapet du fait que la vanne inspiratoire 40, si elle agencée près de l'aval de la chambre 36, interdit de toute façon la remontée trop en amont des gaz expirés.

Au contraire, les gaz expirés par le patient sont recueillis par le circuit expiratoire 14 et sont évacués vers la sortie de gaz expirés 52 au travers d'un débitmètre 122 agencé entre la conduite expiratoire 32 et la chambre expiratoire 48 et au travers de la vanne expiratoire 54.

L'électrovanne 104 de la vanne expiratoire 54 est alors en position 2-3 de sorte qu'il est possible de moduler la pression dans le logement de commande 80 de la vanne expiratoire 54 en agissant sur la vanne proportionnelle 112 ce qui permet de créer, dans le circuit expiratoire 14, une pression d'expiration positive (PEP) de niveau déterminé favorable au traitement de certaines formes d'insuffisance respiratoires. Dans ce cas, un faible débit de fuite, qui s'écoule par la dérivation 42, permet de compenser d'éventuelles fuites au niveau du masque du patient.

Toutefois, le respirateur 10 selon l'invention comporte des moyens pour faciliter, en début de phase expiratoire, l'expiration du patient, notamment au moment où celui-ci doit expirer un maximum de gaz sous un débit important.

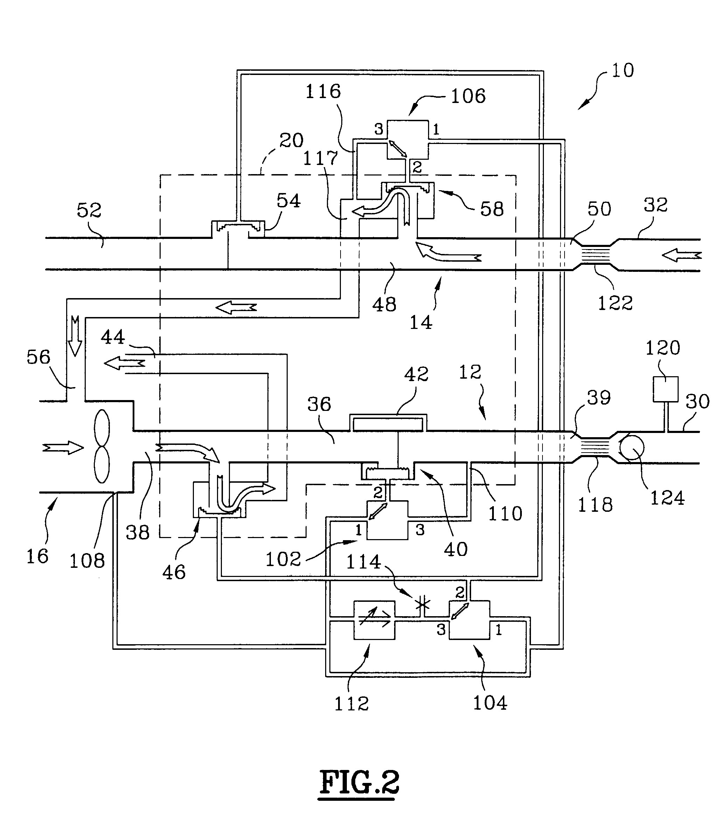

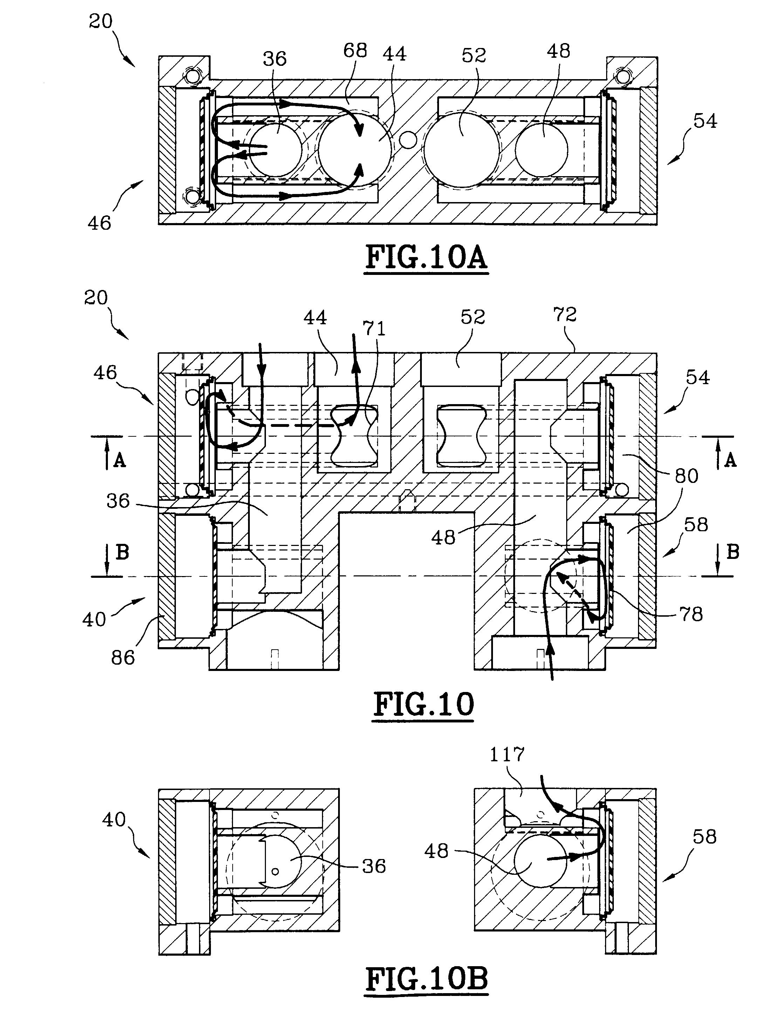

A cet effet, comme on peut le voir sur les figures 2, 10, 10A et 10B, pendant une durée qui peut par exemple varier entre un huitième et un quart de la durée totale de la phase expiratoire, la vanne de dépression 58 est ouverte pour relier la chambre expiratoire 48 à la source de dépression constituée par l'entrée d'aspiration d'air 56 du moto-ventilateur 16.

De la sorte, la pression dans le circuit expiratoire 14 chute et le volume d'air contenu dans le circuit expiratoire 14 est mis en mouvement par une action autre que la simple expiration du patient, ce qui facilite grandement le travail d'expiration de ce dernier.

Une fois la phase expiratoire ainsi amorcée, la vanne de dépression 58 est refermée pour permettre la poursuite de l'expiration au travers la vanne d'expiration 54 ainsi que cela à été vu précédemment.

L'invention peut aussi être mise en oeuvre dans le cas où la vanne d'expiration n'est pas agencée dans le respirateur mais, par exemple, au niveau du masque qui est porté par le patient. Dans ce cas, l'enceinte délimitée par le masque forme une chambre expiratoire et il suffit de relier l'enceinte du masque à une vanne de dépression commandée par le respirateur pour provoquer, dans le masque, une chute de pression favorable à l'amorce de l'expiration.

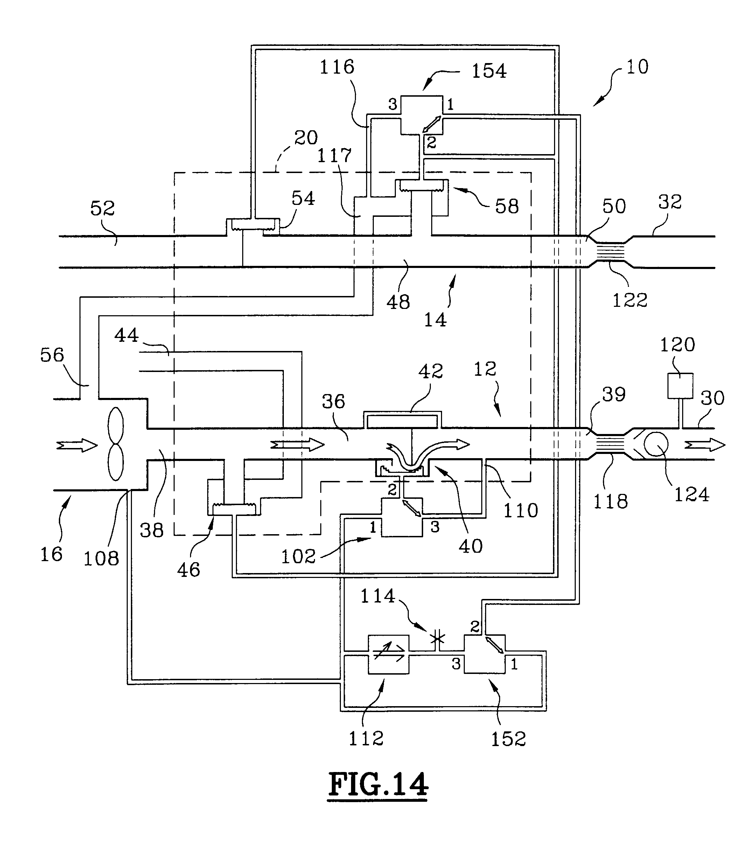

On a représenté sur la figure 14 une variante de réalisation d'un appareil d'assistance respiratoire selon l'invention. Dans cette figure, les éléments identiques ou similaires à ceux décrits précédemment sont désignés par les mêmes chiffres de référence.

Dans ce deuxième mode de réalisation de l'invention, les logements de commande 80 des vannes d'échappement 46, expiratoire 54 et de dépression 58 sont tous commandés simultanément de manière identique. En effet, ils sont chacun reliés à l'entrée 154-2 d'une première électrovanne ou sélecteur 154 qui commande ainsi l'ouverture ou la fermeture des vannes correspondantes.

En effet, l'entrée 154-3 du sélecteur 154 est reliée, par la dérivation 116, à l'aval 117 de la vanne de dépression 58, c'est-à-dire à une source de dépression. Ainsi, lorsque le sélecteur 154 est en position 2-3, il se produit sur chacune des membranes 78 des vannes correspondantes une action positive d'ouverture due à la dépression qui règne alors dans leur logement de commande 80.

Ce dispositif permet notamment d'accélérer l'ouverture de la vanne d'échappement 46 et de la vanne expiratoire 54 dans le but de faciliter encore l'expiration du patient.

Au contraire, l'entrée 154-1 du sélecteur 154 est reliée à l'entrée 152-2 d'une seconde électrovanne dont les entrées 152-1 et 152-3 sont respectivement reliées à la prise de pression 108 en sortie de motoventilateur 16 et à la prise de pression régulée constituée par l'électrovanne proportionnelle 112 et la fuite calibrée 114.

De la sorte, lorsque l'électrovanne formant sélecteur 154 est en position 2-1, les logements de commande 80 des trois vannes 46, 54, 58 reliées au sélecteur 154 sont soumis à une pression qui peut être soit la pression en sortie de turbine, ce qui provoque une fermeture de ces vannes, soit la pression régulée par l'électrovanne proportionnelle 112, auquel cas il est possible de maîtriser le degré d'ouverture de ces trois vannes en jouant sur le réglage de l'électrovanne proportionnelle 112.

Il est à noter que, dans ce mode de réalisation de l'invention, la vanne de dépression 58 est obligatoirement ouverte pendant toute la durée de la phase d'expiration.

Comme cela a été indiqué précédemment, le bloc de distribution 20 est aisément démontable de l'appareil pour en permettre un nettoyage aisé.

Dans ce but, comme on peut le voir à la figure 4, le respirateur 10 selon l'invention comporte un panneau arrière 126 qui est destiné à être agencé en regard de la face arrière 72 du bloc 20 lorsque ce dernier est monté dans l'appareil 10. Ce panneau arrière 126, qui est fixé dans l'appareil comporte, sur une face arrière 128, des moyens qui permettent son raccordement avec, notamment, le moto-ventilateur 16 et les électrovannes 102, 104, 106 de commande des vannes. Sur une face avant 130, le panneau arrière 126 comporte des moyens de raccordement correspondants qui sont susceptibles de coopérer avec des moyens complémentaires portés par la face arrière du bloc 20 et qui permettent de dégager le bloc 20 par un simple coulissement du bloc 20 vers l'avant selon la direction longitudinale.

Ainsi que cela est représenté à la figure 4, les capteurs de débit 118, 122, interposés respectivement dans les circuits inspiratoire 12 et expiratoire 14 sont montés à l'avant du bloc de distribution 20. Les deux capteurs 118, 122 sont réalisés selon une conception identique qui est représentée de manière schématique à la figure 12.

Chaque capteur 118, 122 comporte ainsi un corps externe 132 sensiblement tubulaire et une douille tubulaire 134 qui est engagée selon la direction longitudinale dans un alésage cylindrique correspondant du corps externe 132 et dont une surface cylindrique interne 136 délimite un canal interne longitudinal pour le passage de l'air qui est destiné à être inspiré ou qui a été expiré par le patient.

Une surface cylindrique externe 138 de la douille 134 comporte deux gorges annulaires externes 140 qui sont espacées longitudinalement l'une de l'autre. La surface cylindrique interne 136 de la douille 134 comporte elle aussi deux gorges annulaires internes 142 qui sont agencées longitudinalement en correspondance respectivement avec les gorges externes 140.

Les gorges externes 140 et internes 142 correspondantes sont reliées par des perçages radiaux 144 qui sont répartis sur la circonférence des gorges 140, 142.

Le corps externe 132 comporte deux orifices 146 parallèles d'orientation radiale qui débouchent vers l'intérieur chacun en regard d'une des deux gorges externes 140. Des embouts de raccordement 148 sont destinés à être introduits dans les orifices 146 pour être reliés à des transducteurs du circuit de commande du respirateur 10 qui sont sensibles à la pression.

Par ailleurs, la douille 134 est indexée angulairement pour que les orifices 146 du corps externe 132 ne débouchent pas directement en regard d'un perçage 144 de la douille.

Un corps poreux est interposé dans le canal interne délimité par la douille 134, longitudinalement entre les séries de gorges correspondantes. Ce corps poreux 150 est avantageusement réalisé sous la forme d'une structure en nid d'abeille comportant des cellules qui s'étendent longitudinalement et qui sont ouvertes selon cette direction.

Ainsi, lorsqu'un flux d'air s'écoule dans le canal interne de la douille 134, les transducteurs sont soumis chacun à la pression statique qui règne du côté correspondant du corps poreux 150. De la différence de ces deux pressions, il est possible de déterminer par calcul, après une opération de calibrage, le débit qui s'écoule au travers du débitmètre.

## Revendications

1. Appareil d'assistance respiratoire, du type comportant une source d'air sous pression (16) qui fournit de l'air sous pression destiné à être acheminé, lors d'une phase inspiratoire, vers un patient au travers d'un circuit inspiratoire (12), et du type dans lequel de l'air expiré par le patient au cours d'une phase expiratoire est recueilli par un circuit expiratoire (14),
caractérisé en ce que l'appareil (10) comporte des moyens (58) pour relier le circuit expiratoire (14) à une source de dépression (56) en début de phase expiratoire.

2. Appareil d'assistance respiratoire selon la revendication 1, caractérisé en ce que le circuit expiratoire (14) comporte une chambre expiratoire (48) qui est agencée dans l'appareil, et une conduite expiratoire (32) qui relie la chambre expiratoire (48) au patient, et en ce que la chambre expiratoire (48) est reliée d'une part à une sortie (52) des gaz expirés par une vanne expiratoire (54), et d'autre part à la source de dépression (56) par une vanne de dépression (58).

3. Appareil d'assistance respiratoire selon la revendication 2, caractérisé en ce qu'un capteur de débit (122) est interposé dans le circuit expiratoire (14) entre la conduite expiratoire (32) et la chambre expiratoire (48).

4. Appareil d'assistance respiratoire selon l'une quelconque des revendications précédentes, caractérisé en ce que le circuit inspiratoire (12) comporte une chambre inspiratoire (36) qui est agencée dans l'appareil (10) et dans laquelle débouche la source d'air sous pression (16), et une conduite inspiratoire (30) qui relie la chambre inspiratoire (36) au patient, et en ce que la chambre inspiratoire (36) est reliée d'une part à la conduite inspiratoire (30) au travers d'une vanne inspiratoire (40), et d'autre part à une sortie d'échappement (44) par une vanne d'échappement (46).

5. Appareil d'assistance respiratoire selon la revendication 4 prise en combinaison avec l'une quelconque des revendications 2 ou 3, caractérisé en ce que les vannes inspiratoire (40), expiratoire (54), d'échappement (46), et de dépression (58) sont réalisées sous la forme de vannes à membranes à commande pneumatique, et en ce qu'elles sont agencées dans un bloc de distribution (20) dans lequel sont formées les chambres inspiratoire (36) et expiratoires (48).

6. Appareil d'assistance respiratoire selon la revendication 5, caractérisé en ce que l'une au moins des vannes à commande pneumatique (40, 46, 54, 58) est aménagée sous la forme d'une empreinte (60) creusée dans le bloc de distribution (20), en ce que l'empreinte (60) débouche vers l'extérieur dans une face latérale (62) du bloc (20) et comporte, dans le fond, une enceinte interne (64) et une enceinte périphérique (66) aménagée autour de l'enceinte interne (64), en ce que les deux enceintes (64, 66) sont délimitées l'une de l'autre par une paroi (74) dont une extrémité s'étend vers l'extérieur sous la forme d'un tronçon tubulaire qui se termine par une face annulaire transversale (76) tournée vers l'extérieur et formant siège d'étanchéité, en ce qu'une membrane transversale souple (78) est liée de manière étanche par son bord périphérique (77) à une paroi (94) de l'empreinte (60) du bloc (20), vers l'extérieur par rapport au siège d'étanchéité (76), pour séparer les deux enceintes (64, 66) d'un logement de commande (80), et en ce qu'une surpression dans le logement de commande (80) est susceptible de plaquer la membrane (78) en appui contre le siège d'étanchéité (76) pour isoler de manière étanche l'enceinte interne (64) de l'enceinte périphérique (66) et ainsi fermer la vanne en interrompant la communication entre les deux enceintes (64, 66).

7. Appareil d'assistance respiratoire selon la revendication 6, caractérisé en ce que le logement de commande (80) est délimité vers l'extérieur par une face interne (82) d'un capot distributeur (84), en ce qu'une conduite d'alimentation (96) du logement de commande (80) est aménagée dans le bloc de distribution (20) et débouche dans une surface périphérique (94) de l'empreinte (60), en regard d'une gorge (90) qui est aménagée dans une surface périphérique (88) du capot distributeur (84), et en ce que le capot (84) comporte un canal (98) qui débouche d'une part dans la gorge (90) et d'autre part dans la face avant (82) pour relier la conduite d'alimentation (96) au logement de commande (80).

8. Appareil d'assistance respiratoire selon l'une quelconque des revendications précédentes, caractérisé en ce que le bloc de distribution (20) est monté de manière amovible dans l'appareil (10).

9. Appareil d'assistance respiratoire selon l'une quelconque des revendications précédentes, caractérisé en ce que la source de pression est un moto-ventilateur (16), et en ce que la source de dépression est constituée par une entrée d'aspiration (56) du moto-ventilateur (16).

10. Appareil d'assistance respiratoire selon la revendication 9, caractérisé en ce que la source de pression (16) est un moto-ventilateur de type centrifuge.

11. Appareil d'assistance respiratoire selon l'une quelconque des revendications précédentes, caractérisé en ce que le capteur de débit (118, 122) comporte un corps externe (132) dans lequel est engagée une douille tubulaire (134) qui délimite un canal d'écoulement de l'air, en ce que la douille (134) comporte deux séries de perçages radiaux (144) qui débouchent d'une part vers l'intérieur, respectivement de part et d'autre axialement d'un corps poreux (150) interposé dans le canal d'écoulement, et d'autre part vers l'extérieur respectivement dans deux gorges annulaires (140) aménagées dans une surface cylindrique externe (138) de la douille (134), et en ce que le corps externe (132) comporte deux orifices (146) qui débouchent chacun en regard d'une des gorges (140) et qui sont reliés à des capteurs de pression.

12. Appareil d'assistance respiratoire selon la revendication 11, caractérisé en ce que les orifices (146) de prise de pression du corps externe (132) ne débouchent pas en regard d'un perçage radial (144) de la douille (134).
